(19) Europäisches Patentamt
European Patent Office
Office européen des brevets

(11) **EP 0 947 522 B1**

(12) **FASCICULE DE BREVET EUROPEEN**

(45) Date de publication et mention de la délivrance du brevet:
**06.03.2002 Bulletin 2002/10**

(51) Int Cl.7: **C07H 13/08**

(21) Numéro de dépôt: **99200220.4**

(22) Date de dépôt: **15.11.1994**

(54) **Procédé de préparation de l'oenothéine B à partir d'Epilobium Parviflorum**

Verfahren zur Herstellung von Oenothein-B aus Epilobium Parviflorum

Process for the preparation of oenotheine B from epilobium parviflorum

(84) Etats contractants désignés:
**CH DE FR GB IT LI NL**

(30) Priorité: **16.11.1993 FR 9313623**

(43) Date de publication de la demande:
**06.10.1999 Bulletin 1999/40**

(62) Numéro(s) de document de la (des) demande(s) initiale(s) en application de l'article 76 CBE:
**94402575.8 / 0 657 167**

(73) Titulaire: **Aventis Pharma S.A.**
**92160 Antony (FR)**

(72) Inventeurs:
• **Gourvest, Jean-François**
**77410 Claye-Souilly (FR)**
• **Kasal, Alexander**
**101 00 Prague 10 (CZ)**
• **Lesuisse, Dominique**
**93100 Montreuil (FR)**
• **Teutsch, Jean-Georges**
**93500 Pantin (FR)**

(56) Documents cités:
**EP-A- 0 297 547**

• **DATABASE WPI Section Ch, Week 198729 Derwent Publications Ltd., London, GB; Class B04, AN 1987-201982 XP002128034 & JP 62 129220 A (LEAD CHEM CO LTD), 11 juin 1987 (1987-06-11)**
• **TAKUO OKUDA ET AL: "FRACTIONATION OF PHARMACOLOGICALLY ACTIVE PLANT POLYPHENOLS BY CENTRIFUGAL PARTITION CHROMATOGRAPHY" JOURNAL OF LIQUID CHROMATOGRAPHY,US,NEW YORK, NY, vol. 13, no. 18, page 3637-3650-3650 XP002100162**

**Description**

La présente invention concerne un procédé de préparation de l'oenotheine B à partir d'Epilobium Parviflorum.

Il existe environ 200 espèces différentes d'Epilobium (famille des Onagreaceae). L'Epilobium Parviflorum ainsi que d'autres espèces d'Epilobium sont décrits en médecine traditionnelle comme bénéfiques à toutes sortes de désordres tels que ceux de la vessie, du rein et plus particulièrement de la prostate (A. Hiermann, H. Juan et W. Sametz, J. Ethnopharmacol. 17 (1986) 161-167 ; V.E. TYLER Pharm. Int. (1986) 205).

Les constituants d'Epilobium sont multiples et de structures complexes. Plusieurs analyses ont été tentées à ce jour et mettent en évidence divers types de composés. Toutefois, la composition demeure mal connue.

On a ainsi trouvé la présence de stérols (A. Hiermann , Sci. Pharm. 51 (1985) 39), de triterpènes et de flavonoides (flavonol aglycones et glycosides) (A. Hiermann et K. Mayr, Sci. Pharm. 51 (1983) 158). Récemment, la séparation des flavonols glycosides de l'Epilobium Parviflorum a permis de mettre en évidence la présence de la quercitrine, de la myricitrine, de l'isomyricitrine, d'acide gallique et d'acide chlorogénique, (I. Slacanin, A. Marston, K. Hostettmann, N. Delabays et C. Darbellay J. Chrom. 557 (1991) 391-398).

La demanderesse vient de découvrir que l'Epilobium Parviflorum renfermait en outre un autre composé particulièrement intéressant jamais isolé à ce jour de cette plante, l'oenotheine B.

L'oenotheine B est un tannin oligomérique hydrolysable. Il a été isolé pour la première fois des feuilles d'Oenothera erythrosepala Borbas (T. Hatano et al J. Chem. Soc., Perkin Trans. 1, (1990), 2735 ; 104th Annual Meeting of the Pharmaceutical Society of Japan, Mars 1984, Sendai et Chem. Pharm. Bull. (1989) 37(8) 2269-2271).

L'oenotheine B a été ensuite trouvée dans différentes plantes Onagracées et dans Woodfordia fruticosa (T. Yoshida et al. Chem. Pharm. Bull. 38, 1211 (1990) qui fait partie de la famille des Lythraceae. Enfin, récemment, l'oenotheine B a été isolée à partir du fruit séché de l'Eucalyptus Alba REINW qui fait partie de la famille des Myrtaceae (T. Yoshida et al. Chem. Pharm. Bull. (1992) 40(7) 1750-1754).

Bien que découverte à une époque récente, l'oenotheine B a fait l'objet d'études nombreuses qui ont démontré son intérêt en tant que médicament. L'oenotheine B possède ainsi un certain nombre de propriétés biologiques et pharmacologiques dans le domaine antiviral et antitumoral.

Activité antitumorale :

Lead Chemical Co J62129220 (11/06/87) Pr : 85JP-270494 (30/11/85)
Nippon Kayaku Co J03123793 (27/05/91) Pr : 89JP-258708 (05/10/89)
Ken-ichi Miyamoto et al. Biol. Pharm. Bull. 16(4) 379-387 (1993)
A. Kuramochi-Motegi et al. Biochem. Pharm. 44(10) 1961-1965 (1992)
Yan-Jyu Tsai et al. The Journal of Biological Chemistry 267(20) 14436-14442
Ken-ichi Miyamoto et al. Jpn. J. Cancer Res. 84, 99-103 (1993).

Activité antivirale (Herpes) :

K. Fukuchi etr al. Antiviral Research 11 (1989) 285-298

Activité antivirale (HIV) :

Lead Chemical Co EP-297547 (04/01/89) Pr 87JP-161572 (29/06/87).

C'est lors de l'étude d'un extrait particulier (fraction aqueuse) d'une décoction d'Epilobium Parviflorum, que la demanderesse a observé que le dit extrait répondait de manière significative comme inhibiteur de l'enzyme testostérone 5-$\alpha$-réductase, une enzyme clé dans la biosynthèse de certains androgènes, en particulier la 4,5-$\alpha$-dihydrotestostérone (DHT).

Cette observation a conduit la demanderesse à tenter d'identifier la molécule responsable de cette inhibition. Celle-ci s'est révélée être l'oenothéine B.

En résumé, c'est donc la première fois que l'on isole et identifie l'oenotheine B comme l'un des principes actifs de l'Epilobium Parviflorum.

Et c'est la première fois que l'on détermine que l'oenotheine B agit en tant qu'inhibiteur de l'enzyme testostérone 5-$\alpha$-réductase ce qu'en aucune manière l'art antérieur ne laissait prévoir.

L'oenotheine B présente une activité spécifique en tant qu'inhibiteur de la 5-$\alpha$-réductase. La 5-$\alpha$-réductase est une enzyme responsable de la conversion de la Testostérone en dihydrotestostérone (DHT), plus affine que la Testostérone sur le récepteur des androgènes (RA).

Cette hormone est essentielle chez le mâle, au développement in utéro des organes sexuels externes et ensuite dès la puberté, à l'apparition de certaines caractéristiques sexuelles secondaires (système pileux, visage et corps).

On retrouve la 5-$\alpha$-réductase essentiellement dans la prostate et dans la peau, là où il semble que la Testostérone doive être transformée en DHT pour agir.

Un excès de DHT peut être responsable d'acné ou d'alopécie androgénique, d'hirsutisme chez la femme, et son accumulation au niveau de la prostate, d'hyperplasie bénigne (HBP) ou de cancer de la prostate. On estime que 80 % des hommes de plus de 80 ans présentent une HBP et 70 % des foyers cancéreux.

L'inhibition de cette étape enzymatique dans le métabolisme stéroïdien entraîne une chute du niveau de DHT in vivo. Cette réduction du niveau de DHT a pour effet de réduire la taille de la prostate chez l'homme et donc a un effet bénéfique dans le traitement de l'hyperplasie bénigne de la prostate, du cancer de la prostate, et de certaines maladies androgéno-dépendantes.

Un traitement inhibant la 5-$\alpha$-réductase est donc très utile et ne présente pas les inconvénients d'un antiandrogène qui bloque le récepteur.

Cette propriété inhibitrice de la 5-$\alpha$-réductase rend donc l'oenotheine B apte à être utilisée dans le traitement des troubles liés à l'hyperandrogénie, notamment celui de l'hyperplasie bénigne de la prostate (HBP), du cancer de la prostate, de l'acné, de l'alopécie androgénique, de la séborrhée, ou encore de l'hirsutisme féminin.

La posologie varie en fonction de l'affection à traiter et de la voie d'administration.

Elle peut varier par exemple de 5 à 100 mg/kg et, de préférence, de 10 à 20 mg/kg et par jour chez l'adulte par voie orale.

L'oenotheine B peut donc être employée pour préparer des compositions pharmaceutiques la contenant à titre de principe actif et l'invention a aussi pour objet lesdites compositions pharmaceutiques.

L'oenotheine B selon l'invention peut être utilisée par voie digestive, parentérale ou locale, par exemple par voie transdermique. Elle peut être prescrite sous forme de comprimés simples ou dragéifiés, de gélules, de granulés, de suppositoires, de préparations injectables, d'ovules, de pommades, de crèmes, de gels, de patchs, lesquels sont préparés selon les méthodes usuelles.

Le principe actif peut y être incorporé à des excipients habituellement employés dans ces compositions pharmaceutiques tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou emulsifiants, les conservateurs.

L'invention a pour objet un procédé de préparation d'oenotheine B à partir d'Epilobium Parviflorum caractérisé en ce qu'on effectue successivement :

a) une extraction en additionnant soit de l'eau soit un mélange : solvants miscibles à l'eau/eau, à des échantillons séchés d'Epilobium Parviflorum, puis

b) une filtration et, le cas échéant, une concentration sous pression réduite jusqu'à évaporation du solvant miscible à l'eau, puis

c) soit une lyophilisation de la phase aqueuse, soit une extraction avec un solvant polaire que l'on évapore jusqu'à obtention d'un extrait sec, et

d) une purification du lyophilisat ou de l'extrait sec, soit par HPLC en phase inverse, soit par colonne de gels polymériques.

Le procédé de préparation consiste donc essentiellement en une extraction suivie d'une purification dont les mises en oeuvre sont effectuées comme suit :

Extraction

De l'eau ou un mélange d'eau et de solvants miscibles à l'eau tels que les alcools choisis parmi le méthanol, l'éthanol, l'isopropanol, le butanol, ou bien l'acétone sont ajoutés à des échantillons séchés d'Epilobium Parviflorum. Il s'agit de préférence de feuilles d'Epilobium Parviflorum.

Le solvant miscible à l'eau préféré est l'acétone.

Après agitation et filtration, le filtrat est le cas échéant concentré sous pression réduite jusqu'à évaporation du solvant miscible à l'eau. La phase aqueuse restante est soit lyophilisée, soit extraite avec différents solvants organiques polaires connus de l'homme du métier tels que par exemple l'éther diéthylique, l'acétate d'éthyle ou le butanol. Il s'agit de préférence du butanol. Ces fractions sont ensuite évaporées sous pression réduite à une température n'excédant pas 50°C, jusqu'à l'obtention d'un extrait sec.

Purification

La purification du principe actif s'effectue soit par passage sur une colonne de gels polymériques, soit par HPLC en phase inverse.

Purification par colonne de gels polymériques :

On dissout l'extrait sec ou le lyophilisat obtenus précédemment dans de l'eau distillée et on verse sur une colonne de gels polymériques, par exemple du type DIA ION HP20, SEPHADEX LH20, TOYOPEARL HW40.

L'élution s'effectue avec de l'eau distillée puis un mélange alcool/eau distillée en proportion croissante. La fraction contenant l'oenotheine B est identifiée par HPLC, puis lyophilisée. L'alcool utilisé est de préférence le méthanol.

Purification par HPLC en phase inverse

On dissout l'extrait sec ou le lyophilisat obtenu précédemment dans de l'eau distillée puis on le purifie par HPLC en phase inverse. On élue avec un gradient composé d'un mélange de solvant polaire et hydrophile tel que le mélange acétonitrile/eau à pH acide en utilisant un acide tel que l'acide trifluoroacétique (TFA). La fraction contenant l'oenotheine est par la suite isolée en fonction du temps de rétention et de son spectre obtenu par analyse au détecteur à barette de diodes (210-500 nm) ou au détecteur UV. Après une seconde purification éventuelle, on procède à une lyophilisation du produit attendu.

L'invention a plus particulièrement pour objet un procédé de préparation d'oenotheine B à partir d'Epilobium Parviflorum tel que décrit précédemment caractérisé en ce que l'extraction s'effectue en additionnant un mélange acétone/eau à des échantillons séchés d'Epilobium Parviflorum.

Les exemples suivants illustrent l'invention sans toutefois la limiter.

**Exemple 1 : Extraction des feuilles et branches d'Epilobium Parviflorum avec le mélange acétone/eau et obtention de l'oenotheine B brute.**

Dans un réacteur de 100 litres, on introduit 1275 g de feuilles et branches d'Epilobium Parviflorum séchées et brisées. On y additionne 20 litres d'un mélange acétone-eau 7-3. On agite le mélange au moyen d'un agitateur mécanique durant 60 heures. Le mélange est filtré et les feuilles sont rincées avec 4 litres d'acétone. Le filtrat est concentré sous pression réduite à 35°C jusqu'à un volume d'environ 6 litres de façon à ce que l'acétone soit évaporée. La phase aqueuse restante est extraite successivement avec 2 x 1 litre d'éther diéthylique, 2 x 1 litre d'acétate d'éthyle et 2 x 0,5 litre de butanol. On obtient, après évaporation de ces deux premières fractions sous pression réduite à une température de 35°C, respectivement 100 g de résine noire et 10 g d'une mousse friable marron et après évaporation de la 3ème fraction sous pression très réduite, à une température de 50°C, 45 g d'une résine noire. Cette dernière fraction est la plus riche en oenotheine B.

**Exemple 2 :** Extraction des feuilles et branches d'Epilobium Parviflorum avec de l'eau et obtention de l'oenotheine B brute.

20 g de feuilles et branches d'Epilobium Parviflorum concassés sont couverts par 350 ml d'eau à 65°C. On laisse ensuite le mélange sous agitation mécanique durant 20 heures à température ambiante. On filtre le mélange et rince les feuilles avec 100 ml d'eau distillée. On obtient ainsi 450 ml d'une solution noire limpide. Une lyophilisation de 50 ml de cette solution fournit 500 mg d'une poudre vert pâle riche en produit attendu.

**Exemple 3 : Identification et purification et de l'oenotheine B**

Le lyophilisat préparé selon l'exemple 2 est mis en solution (2 mg/ml d'eau distillée). 10 ml (20 mg) sont injectés sur une colonne préparative de type NOVAPACK™ (C18 6μ 7,8 x 300 mm). L'élution s'effectue par un gradient de solvant à un débit de 5 ml/min dans les conditions suivantes (pourcentages en volumes) :

0-2 mn échantillon
2-7 mn 100 % eau à 0,1 % v/v d'acide trifluoroacétique (TFA),
7-57 mn Gradient linéaire de 0 à 10 % v/v d'acétonitrile à 0,1 % v/v TFA, 57-77 mn Gradient linéaire de 10 à 25 % v/v d'acétonitrile à 0,1 % v/v TFA,
77-97 mn Gradient linéaire de 25 à 90 % v/v d'acétonitrile à 0,1 % v/v TFA.

La détection s'effectue via une barette de diodes à des longueurs d'ondes s'échelonnant de 210 nm à 400 nm.

Sur les 43 fractions récoltées, une seule possède une activité vis-à-vis de la 5-α-réductase, le temps de rétention est compris entre 36 et 38 mn.

Après contrôle de pureté, cette fraction est lyophilisée. Le produit attendu est immédiatement analysé par RMN et spectrométrie de masse. (Les analyses RMN et masse de cette fraction sont comparables à celles décrites dans la littérature : T. HATANO et al. J. Chem. Soc. Perkin Trans 1 (1990) 2735-2743).

RMN

250 MHz,
Solvant : acétone-$d_6$, à 45°C. Ref $SiMe_4$, δ (ppm), J (Hz)

| α glucose | β glucose |
|---|---|
| H-1 6,20 d J=3,5 | H-1 4,42 d J=8 |
| H-2 5,87 dd J=3,5-10,5 | H-2 5,16 dd J=8-9,5 |
| H-3 6,11 dd J=10,5 | H-3 5,45 t J=9,5 |
| H-4 5,59 t J=10,5 | H-4 4,90 t J=9,5 |
| H-5 4,57 dd J=6-10 | H-5 4,13 dd J=5-9,5 |
| H-6 5,24 dd J=6-13 | H-6 5,01 dd J=5-13 |
| 3,63 d J=13 | 3,84 d J=13 |

voir formule Exemple 4.

Spectre de Masse

m/z MALDI (Matrix Assited Laser Desorption Ionisation)

1569 [(M+H)]$^+$
1591 [(M+Na)]$^+$
1607 [(M+K)]$^+$

**Exemple 4 : Purification de l'oenotheine B**

On utilise une colonne préparative de microbondapack (C18 10μ, longueur 50 cm, diamètre 2 cm (1 pouce)). On injecte 1 g de lyophilisat, préparé selon l'exemple 2, dissous dans 50 ml d'eau. On élue avec un mélange éluant eau-acétonitrile à 0,1 % d'acide trifluoroacétique. On réalise une montée en gradient de la concentration en acétonitrile de 15 % v/v en 30 minutes. On détecte les produits au moyen d'un détecteur UV (longueur d'onde 305 nm). Le produit attendu sort avec un temps de rétention de 27 minutes. Après lyophilisation des fractions, on récupère environ 35 mg de produit attendu pur sous la forme d'une poudre beige.

UV

MeOH/HCl 0,1 N        218 nM (∈= 120000) et 266 nM (∈= 55000)
MeOH               218 nM (log∈ = 5,09) et 265 nM (log∈ = 4,77)
MeOH/NaOH 0,1 N    285 nM (∈= 33000) et infl. 315 et 473 nM

Modification non réversible.

Dichroïsme circulaire

MeOH/Hcl 0,1 N     Max. 219 nM (θ) = + 3,8 105 infl. 236 nM (θ) = + 1,7 105 Max. 259 nM (θ) = - 4,5 104 infl. 282 nM (θ) = + 9,5 104

RMN

à 400 (C 13) MHz

Solvant :         $D_2O$
Température :    297 K pour le proton
                    318 K pour le carbone 13.
Référence :     TSF à 0 ppm
Abréviations :   δ (ppm) déplacement chimique en ppm
                    J (Hz) constante de couplage en Hz.

Multiplicités :

s      singulet
sl     singulet large
d      doublet
dd    doublet de doublet
t      triplet

OENOTHEINE B

| Résidu | N° | δ(ppm)$^1$H | Multiplicité (proton) | $^3$J(Hz) proton | δ(ppm)$^{13}$C |
|---|---|---|---|---|---|
| Glucose α | 1 | 5,67 | d | 3,5 | 92,8 |
| | 2 | 4,80 | dd | 3,5-10 | 77,4 |
| | 3 | 5,87 | dd | 10 | 73,7 |
| | 4 | 5,61 | t | 10 | 72,7 |
| | 5 | 4,70 | dd | 6,5-10 | 70,7 |
| | 6 | 4,00 | d | 13,5 | 66,5 |
| | | 5,34 | dd | 6,5-13,5 | |
| Glucose β | 1 | 4,74 | d | 8 | 97,9 |
| | 2 | 5,27 | dd | 10-8 | 77,8 |
| | 3 | 5,51 | t | 10 | 75,5 |
| | 4 | 5,15 | t | 10 | 74,1 |
| | 5 | 4,24 | dd | 10-5 | 74,2 |
| | 6 | 4,04 | d | 13,5 | 67,0 |
| | | 4,87 | dd | 5-13,5 | |
| Galloyl α (Gα) | 1 | - | | | 124,4 |
| | 2-6 | 6,90 | s | - | 112,4 |
| | 3-5 | - | | | 147,6 |
| | 4 | - | | | 140,9 |
| | C=0 | - | | | 171,6 |
| Galloyl β (Gβ) | 1 | - | | | 122,7 |
| | 2-6 | 7,21 | s | - | 113,6 |
| | 3-5 | - | | | 147,6 |
| | 4 | - | | | 141,8 |
| | C=0 | - | | | 169,0 |
| Valoneoyl I Cycle A | 1 | - | | | 116,6 |
| | 2 | - | | | 126,0b |
| | 3 | 6,73 | s | | 110,4a |
| | 4 | - | | | 148,0 |
| | 5 | - | | | 138,3 |
| | 6 | - | | | 146,4c |
| | C=O | - | | | 172,0 |
| Valoneoyl I Cycle B | 1 | - | | | 122,9 |
| | 2 | - | | | 128,2b |
| | 3 | 6,49 | sl | | 116,5 |
| | 4 | - | | | 149,5 |
| | 5 | - | | | 144,2 |
| | 6 | - | | | 146,6c |
| | C=O | - | | | 171,8 |
| Valoneoyl I Cycle C | 1 | - | | | 120,1 |
| | 2 | - | | | 141,6b |
| | 3 | - | | | 129,3b |
| | 4 | - | | | 141,3 |
| | 5 | - | | | 144,9 |
| | 6 | 6,55 | sl | | 112,1 |
| | C=O | - | | | 171,5 |
| Valoneoyl II Cycle A' | 1 | - | | | 118,1 |
| | 2 | - | | | 128,1b |
| | 3 | 6,63 | s | | 110,3a |

(suite)

| Résidu | N° | $\delta$(ppm)$^1$H | Multiplicité (proton) | $^3$J(Hz) proton | $\delta$(ppm)$^{13}$C |
|---|---|---|---|---|---|
| | 4 | - | | | 148,0 |
| | 5 | - | | | 139,2 |
| | 6 | - | | | 146,8c |
| | C=O | - | | | 172,6 |
| Valoneoyl II Cycle B' | 1 | - | | | 119,5 |
| | 2 | - | | | 128,5b |
| | 3 | 6,52 | s | | 108,7 |
| | 4 | - | | | 149,6 |
| | 5 | - | | | 138,6 |
| | 6 | - | | | 147,2c |
| | C=O | - | | | 172,3 |
| Valoneoyl II Cycle C' | 1 | - | | | 118,4 |
| | 2 | - | | | 137,5 |
| | 3 | - | | | 131,6b |
| | 4 | - | | | 142,6 |
| | 5 | - | | | 145,6 |
| | 6 | 6,81 | s | | 113,0 |
| | C=O | - | | | 170,1 |

a - les attributions peuvent être inversées entre-elles
b - les attributions peuvent être inversées entre-elles
c - les attributions peuvent être inversées entre-elles.

## SPECTROMETRIE DE MASSE

### Partie expérimentale

- L'instrument utilisé pour cette étude est un spectromètre triple secteurs et double focalisation "VG Autospec E" (Fison Instrument).
- La masse moléculaire de l'échantillon est déterminée sous ionisation LSIMS (Liquid Secondary Mass Spectrometry) en mode positif et négatif. L'échantillon est préalablement dissous dans une matrice nommée "Magic Ballet/ TFA" avant d'être bombardé par un faisceau d'ions primaires provenant d'un canon Cs$^+$. Ce faisceau est accéléré sous une tension de 30 KV et a un débit de 2 à 3 $\mu$ ampère.
- Les ions secondaires (échantillon) sont accélérés sous une tension de 8 KV.
- L'étude de la fragmentation est faite par balayages liés (Linked scam) selon le mode B/E = Cte. Pour cela l'ion "parent" est fragmenté par collision dans une cellule située en première région libre de champ (1 FFR). Le gaz de collision introduit dans cette cellule est de l'air dont la pression est celle qui est nécessaire pour réduire de moitié l'intensité initiale de l'ion "parent".
- Cette étude est faite en deux temps :

    a) Fragmentation de l'ion quasimoléculaire (MH$^+$)
    b) Fragmentations successives de chacun des ions obtenus précédemment.

### Résultats :

A) LSIMS mode positif
    MH$^+$ = 1569 Da
B) LSIMS mode négatif

(M-H)⁻ = 1567 Da

C) <u>Fragmentation de l'ion MH⁺ = 1569 Da</u>

Les principaux ions observés dans ce spectre sont : 1399⁺ - 1237⁺ - 767+ - 453⁺

D) <u>Fragmentation de l'ion 1237⁺</u>

Les ions principaux observés dans ce spectre sont : 1085⁺ - 767⁺ - 453⁺

E) <u>Fragmentation de l'ion 767⁺</u>

749⁺ - 615⁺ - 471⁺ - 453⁺ - 315⁺ - 152⁺

F) <u>Fragmentation de l'ion 453⁺</u>      Spectre peu caractéristique faisant surtout ressortir l'ion 435 Da (déshydratation).

## Exemple 5 : Purification de l'oenotheine B

710 mg de la résine noire, obtenue selon l'exemple 1, en concentrant la fraction butanolique, sont dissous dans 5 ml d'eau distillée et versés sur une colonne de résine DIA ION HP20 (20 cm x 2 cm).

On élue avec 200 ml d'eau distillée, et ensuite 200 ml d'un mélange eau distillée-méthanol 80-20. Cette deuxième fraction est lyophilisée pour fournir 58 mg d'Oenothéine B sous la forme d'un solide beige pulvérulent. D'autres fractions contiennent également de l'oenothéine B. Les analyses RMN, UV, DC et de spectrométrie de masse sont identiques à celles décrites dans l'exemple 4.

## Etude pharmacologique de l'oenotheine B

Le test de mesure de l'activité 5-$\alpha$-réductase se fait in-vitro en incubant l'enzyme et son substrat (la Testostérone) et en mesurant par chromatographie HPLC la quantité de métabolites 5-$\alpha$-réduits formés (dihydrotestostérone (DHT) et 5-$\alpha$-androstane-diol).

On utilise des homogénats de prostates humaines obtenues par prostatectomies radicales pour cause d'hyperplasie bénigne de la prostate (HBP).

<u>Méthode</u>

<u>Préparation de l'homogénat :</u>

La prostate est recueillie dès la sortie du bloc opératoire et emballée dans du papier aluminium placé dans un récipient rempli de glace carbonique. Au laboratoire, si l'homogénat n'est pas préparé immédiatement, la prostate peut être conservée à -80°C.

On émince la prostate sur de la glace en très petits morceaux, puis on broie dans un milieu A (20 mM de phosphate de potassium, pH 6,5 ; 0,32 M de sucrose ; 1 mM de dithiotréitol (DTT) ; 50 $\mu$M de sel de sodium hydraté de triphosphopyridine nucléotide (NADPH), au polytron puis au potter verre-verre. Après centrifugation à 140000 g pendant 60 mn à 4°C, on remet en suspension dans 10 à 20 ml de milieu B (20 nM de phosphate de potassium, pH 6,5 ; 20 % de glycérol ; 1 mM de dithiotréïtol). On répartit dans des tubes Nunc et on conserve à -80°C.

On effectue un dosage des protéines sur un peu d'homogénat pour son utilisation future dans le test et on s'assure ainsi d'avoir au moins 10 mg de protéines/ml d'homogénat.

<u>Mesure de l'activité 5-$\alpha$-réductase :</u>

L'incubation se fait à pH 5,5 à raison de 1 ml de milieu/tube, en présence de citrate (milieu normal de la prostate).

1) Préparation du substrat (S) c'est-à-dire du mélange de Testostérone "froide" et de Testostérone "tritiée" avec une dilution isotopique de 100 dans un tampon citrate tri-sodique pH 5,0 :

a) tampon citrate tri-sodique 40 mM : On pèse 11,76 g de citrate-3Na, 2H₂O (Merk ref. Art 6448) que l'on dissout dans 1 litre d'eau distillée (Milli-Q) et on ajuste à pH 5,0.

b) mélange de Testostérone "froide" et de Testostérone "tritiée" avec une dilution isotopique de 100 : Pour obtenir une solution de testostérone de 1 mM, on pèse 2,88 mg de Testostérone "froide" que l'on dissout dans 10 ml d'éthanol. Cette solution est diluée au 0,99/1000 soit 9,9 $\mu$l dans 10 ml de tampon citrate (= solution 10⁻⁶M). On ajoute 9 $\mu$l de Testostérone-³H (NET-370) à 10 ml de la solution précédente (= dilution isotopique de 100). Le substrat (S) est prêt.

2) Préparation du mélange Enzyme +DTT +NADPH

    a) Tampon phosphate de potassium, 40 mM, pH 6,5 :
        On pèse 5,44 g de $KH_2PO_4$ (Riedel de Haen ref. 30407) que l'on dissout dans 1 l d'eau distillée (Milli-Q) et on ajuste à pH 6,5.
    b) DTT 1 mM (Sigma)
        On pèse 3,1 mg de DTT et on dissout dans 1 ml de tampon phosphate.
    c) NADPH 500 μM (Sigma)
        On pèse 8,33 mg de NADPH et on dissout dans 1 ml de tampon phosphate.
    d) Mélange enzyme+DTT+NADPH (E) :
        Pour un essai, on mélange à 4°C, 0,5 mg de protéines de l'homogénat de prostate, 50 μl de DTT et 50 μl de NADPH. On complète à 500 μl avec le tampon phosphate.

3) <u>Incubation</u> :
    On prépare autant de tubes que d'essais. Dans chaque tube, on met 500 μl de (S) et 10 μl d'inhibiteur à la concentration voulue (ou d'éthanol pour les témoins).
    On chauffe au bain-marie les tubes contenant le mélange (S) et la fiole contenant le mélange (E) à préincuber pendant 3 à 5 mn.
    Pour démarrer la réaction, on transfère rapidement 500 μl de E dans chaque tube contenant S + l'inhibiteur et on incube à 37°C avec une légère agitation pendant 30 mn. La réaction est stoppée à la fin de l'incubation en mettant les tubes dans de la glace.

4) Extraction
    Aussitôt la réaction stoppée, on met 2 ml d'acétate d'éthyle dans chaque tube et on agite au Multi-tubes vortexer pendant 1 mn. On laisse décanter pendant 10 mn puis on récupère dans d'autres tubes 1,6 ml de la phase organique (supérieure) de l'extrait (= 80 %). Après évaporation totale de l'acétate d'éthyle, on ajoute dans chaque tube 800 μl de la phase mobile qui servira dans l'HPLC.

5) Chromatographie HPLC :
    La mesure des taux de métabolites produits au cours de l'incubation est effectuée en séparant les composés formés par chromatographie liquide à haute performance (HPLC) en phase inverse. On utilise une colonne ODS-Hypersil (diamètre des particules = 5 μm) et une phase mobile composée de $MeOH/THF/H_2O$ dans un rapport de 45/15/40. L'extrait recueilli après extraction est injecté automatiquement au niveau de la chaine HPLC et grâce au système de mesure de radioactivité en ligne (Berthold), on n'apprécie que les métabolites radioactifs, donc formés à partir de la Testostérone marquée du substrat. Cette mesure est très sensible et élimine tous les produits endogènes éventuels.

<u>Expression des résultats et méthodes de calcul</u> :

Les taux de métabolites formés sont calculés par rapport à une courbe étalon rentrée dans le logiciel MT2 de l'HPLC. Au niveau du MT2, ils sortent en pmoles/μl injectés. Des courbes étalons existent pour la testostérone, la DHT et l'androstane-diol, les deux métabolites 5-α-réduits de la testostérone.

Les taux sont exprimés en moles de produits formés/mg de protéines d'homogénat, chaque essai étant effectué en double, et on calcule le pourcentage d'activité 5-α-réductase résiduelle par rapport aux essais témoins :

$$5\ \% = \frac{\text{Taux de produits formés en présence d'inhibiteur} \times 100}{\text{Taux de produits formés pour les témoins}}$$

<u>Résultat</u>

La $CI_{50}$ d'inhibition de l'activité 5-α-réductase du témoin pour l'oenotheine B est de $2,2.10^{-5}$M.

**Revendications**

**1.** Un procédé de préparation d'oenotheine B à partir d'Epilobium Parviflorum **caractérisé en ce qu'**on effectue successivement,

    a) une extraction en additionnant soit de l'eau soit un mélange : solvants miscibles à l'eau/eau, à des échantillons séchés d'Epilobium Parviflorum, puis

b) une filtration et le cas échéant une concentration sous pression réduite jusqu'à évaporation totale du solvant miscible à l'eau, puis

c) soit une lyophilisation de la phase aqueuse, soit une extraction avec un solvant polaire que l'on évapore jusqu'à obtention d'un extrait sec, et

d) une purification du lyophilisat ou de l'extrait sec, soit par HPLC en phase inverse soit par colonne de gels polymériques.

2. Procédé selon la revendication 1, **caractérisé en ce que** l'extraction s'effectue en additionnant un mélange acétone/eau à des échantillons séchés d'Epilobium Parviflorum.

**Claims**

1. A process for the preparation of oenothein B from Epilobium Parviflorum **characterized in that** the following are successively carried out,

a) extraction by adding either water or a mixture: solvents miscible with water/water, to dry samples of Epilobium Parviflorum, then

b) filtration and if appropriate concentration under reduced pressure until total evaporation of the solvent miscible with water, then

c) either lyophilization of the aqueous phase, or extraction with a polar solvent which is evaporated until a dry extract is obtained, and

d) purification of the lyophilisate or of the dry extract, either by reversed phase HPLC or by polymer gel column.

2. Process according to claim 1, **characterized in that** the extraction is carried out by adding an acetone/water mixture to dry samples of Epilobium Parviflorum.

**Patentansprüche**

1. Ein Verfahren zur Herstellung von Oenothein B aus Epilobium Parviflorum, **dadurch gekennzeichnet, dass** man nacheinander ausführt:

a) eine Extraktion, indem man entweder Wasser oder ein Gemisch: wassermischbare Lösungsmittel/Wasser zu getrockneten Proben von Epilobium Parviflorum zugibt, dann

b) eine Filtration und gegebenenfalls ein Konzentrieren unter vermindertem Druck bis zum vollständigen Verdampfen des wassermischbaren Lösungsmittels, dann

c) entweder eine Gefriertrocknung der wässrigen Phase oder eine Extraktion mit einem polaren Lösungsmittel, das man bis zum Erhalt eines trockenen Extraktes verdampft, und

d) eine Reinigung des gefriergetrockneten Produkts oder des trockenen Extraktes entweder durch Inversed Phase HPLC oder durch eine Säule mit Polymergelen.

2. Verfahren gemäß Anspruch 1, **dadurch gekennzeichnet, dass** die Extraktion erfolgt, indem man getrockneten Proben von Epilobium Parviflorum ein Gemisch Aceton/Wasser zugibt.